# EUROPEAN PATENT APPLICATION

(11) **EP 2 918 266 A1**
(43) Date of publication of application: **16.09.2015**
(21) Application number: 14158948.1
(22) Date of filing: 11.03.2014
(51) Int. Cl.: A61K 9/08, A61P 25/16, A61P 5/06, A61P 25/14, A61P 25/26, A61P 25/28, A61K 31/198, A61K 31/4045, A61K 31/428, A61K 31/506

(54) **Composition comprising a dopamine agonist and an L-DOPA derivative for treating Parkinson's disease**

(71) Applicant: CDRD Berolina AB, 23335 Svedala (SE)
(72) Inventor: Alken, Rudolf-Giesbert, 23335 Svedala (SE); Schneider, Frank, 12437 Berlin (DE)
(74) Representative: Müller & Schubert

(57) **Abstract**

Disclosed is a pharmaceutical composition comprising
i) a dopamine agonist and
ii) a L-DOPA derivative

in combination in form of a liquid preparation.

The pharmaceutical composition comprises a dopamine agonist selected from the group comprising apomorphine, ropinirole, rotigotine, pramipexole, and piribedile and a L-DOPA derivative selected from L-DOPA, selectively and/ or partially deuterated L-DOPA derivatives, as well as physiologically acceptable salts of the aforementioned L-DOPA derivatives.

The pharmaceutical composition is provided in a form of a parental application selected from infusions, injections or an application via a gastric tube.

The pharmaceutical composition is used for the treatment of Parkinson's disease, of restless leg syndrome, of dystonia, for inhibiting prolactin secretion, for stimulating the release of growth hormones, for the treatment of neurological symptoms of chronic manganese intoxication, of amyotrophic lateral scleroses and of multiple system atrophy.

## Description

The present invention relates to a pharmaceutical composition comprising a dopamine agonist and a L-DOPA derivative in a combination of a defined ratio for the treatment of Parkinson's disease.

Parkinson's disease is a neurodegenerative disease with a slow progressive course characterized by different symptoms and signs that may be present or develop during the progression of disease. Core symptoms are bradykinesia and at least one of the following, namely resting tremor, muscular rigidity and postural reflex impairment. Other symptoms that may occur during the disease progression are autonomic disturbances, sleep disturbances, disturbances in the sense of smell or sense of temperature as well as depressive symptoms and cognitive dysfunctions.

The improvement of the impaired dopaminergic neurotransmission by administration of L-DOPA is the backbone of the current pharmacotherapy. Patients with advanced Parkinson's disease require higher doses of dopaminergics which is limited by motor complications, like fluctuations and involuntarily movements (described as levodopa induced dyskinesia, LIDs). Fluctuations might be due to the shorter striatal persistence (half life) of dopamine especially in advanced Parkinson's disease patients. A clinical established approach to prolong striatal dopamine persistence is the co-administration of MAO-B inhibitors which block the main metabolic breakdown route of dopamine. The induction of LIDs is associated in many patients with higher CNS dopamine levels generated by large L-DOPA doses.

Apomorphine is a derivative of morphine but does not share the major characteristics of morphine such as soothing the pain, acting euphoric or loss of effectiveness. Apomorphine is a non-selective dopamine agonist which activates both D₁-like and D₂-like receptors and is currently used in the treatment of Parkinson's disease. Apomorphine is used for the treatment of Parkinson patients with insufficient control of motor fluctuations despite individually optimized L-DOPA (L-DOPA + DOPA decarboxylase inhibitor) and /or dopamine agonist therapy in the form of a subcutaneous injection or as a permanent infusion.

Three solutions currently known using apomorphine for intermittent subcutaneous injections are marketed under different trademarks (APO-go^{®} PFS, APO-go^{®} ampoules and Apokyn^{®}). But these formulations cause injection site reactions such as nodulation and the formation of discoloured spots due to their low pH (range 3.0-4.0).

The WO-A 2013/007381 describes a new formulation with apomorphine as active substance wherein the pH is greater than 4 in order to prevent any reactions at the site of injection.

The current therapy of late Parkinson's disease with subcutaneous infusion of apomorphine allows a dose reduction but requires the additional administration of oral dopaminergics to control motor fluctuations. A daily dose of infused apomorphine of 72.00 ± 21.38 mg reduced the does of oral L-DOPA from 989.4 ± 420.1 mg/day to 663.8 ± 403.2 mg/day (Ruiz et al., Mov Disord, 2008, 23(8): 1130-1136). Further a reduction of 55% of L-DOPA was reported by a mean daily dose of apomorphine of 75.2 mg (Katzenschlager et al., 2005, Mov Disord, 20(2): 151-157).

To compare the effects of changes in antiparkinsonian medications, the daily dopaminergic treatment can be calculated as levodopa equivalent dose (LED) using the method decribed by Deuschl et al. (N Engl J Med 2006;355: 898). A 10 mg daily dose of apomorphine is equivalent to standard levodopa dose of 100 mg (LED = 100 mg). A 100 mg dose of levodopa accounts for 100 mg LED.

In addition, levodopa is administered in combination with active additives in pharmaceuticals. Combinations of levodopa are used with peripheral decarboxylase inhibitors, with inhibitors of the enzyme catechol-O-methyltransferase (COMT), with inhibitors of the enzyme monoamine oxidase (MAO) and with dopamine β-hydroxylase inhibitors.

In this connection, the decarboxylase inhibitors used are, for example: D,L-serine 2-(2,3,4-trihydroxybenzyl) hydrazide (benserazide), (-)-L-α-hydrazino3,4-dihydroxy-a-methylhydrocinnamic acid (carbidopa), L-serine-2-(2,3,4-trihydroxybenzyl) hydrazide, glycine-2-(2, 3, 4-trihydroxybenzyl) hydrazide and L-tyrosine-2-(2, 3, 4-trihydroxybenzyl) hydrazide. Examples of combination preparations of levodopa and decarboxylase inhibitors include, among others: Madopar^{®} (levodopa and benserazide hydrochloride) as well as Nacom^{®} (levodopa and carbidopa).

In Duodopa^{®}-pumps a solution of L-DOPA/Carbidopa is used for continuous administration into the small intestine of Parkinson patients. The treatment with Duodopa is used in patients with advanced Parkinson's disease who do not have satisfactory control of severe, disabling motor symptoms with available combinations of medications for Parkinson's disease. The treatment requires a surgery to insert a tube directly into the upper small intestine. Before surgery, a temporary tube through the nose into the small intestine will be used to control response and adjust the dose. Most patients on Duodopa don't need additional oral L-DOPA administration. But the limitations of the gastric transport barrier remain.

Further, at high doses of Duodopa, dyskinesia is present, which is possibly due to the formation of norepinephrine an unavoidable metabolite of L-DOPA and alpha receptor agonist. Alpha blockers have been shown to reduce L-DOPA induced dyskinesia (LIDs) (Lewitt, 2012, Transl Neurodegener, 1(1): 4). Currently there are different pharmacological means under development to treat existing LIDs.

α,β,β-D3-L-DOPA exhibited higher longer-lasting striatal dopamine levels than L-DOPA. Correspondingly to the increased availability of dopamine in the striatum, α,β,β-D3-L-DOPA showed improved motor activity compared to L-DOPA in several Parkinson models (Malmlöf et al., Exp Neurol, 2008, 538-542; Malmlöf et al., Exp Neurol, 2010, 225: 408-415). The equi-effective dose of α,β,β-D3-L-DOPA compared to L-DOPA was about 60%. The observed longer striatal persistence of dopamine allowed the assumption that fluctuations might be reduced as well.

S/S-2-amino-2,3-dideutero-3-(3,4-dihydroxyphenyl) propionic acid (α,β-D2-L-DOPA) and L-2-Amino-2,3,3-trideutero-3-(3,4-dihydroxyphenyl) propionic acid (α,β,β-D3-L-DOPA) were shown to increase and prolong the output of striatal dopamine significantly more than L-DOPA (WO-A 2004/056724 and WO-A 2007/093450).

The highest striatal dopamine concentrations were found after administration of α,β-D2-L-DOPA. Those dopamine levels were even higher than those after the administration of the triple-deuterated α,β,β-D3-L-DOPA which included the same deuterated positions as the double deuterated L-DOPA.

At the equi-effective dose (same striatal dopamine levels and same motor effect as L-DOPA), α,β,β-D3-L-DOPA caused significant less dyskinesia than L-DOPA (Malmlöf et al., Exp Neurol, 2010, 225: 408-415).

The problem to be solved according to the invention is to overcome the drawbacks described in the literature and to provide a new pharmaceutical composition according to the main claim for the treatment of Parkinson's disease.

As used herein and in the context of the present invention the meaning of "deuterated" is extended to partially or completely deuterated compounds. "Completely deuterated" compounds are compounds in which at least 98 mol% deuterium are present in the respective position within the chemical compound. The deviation to 100 Mol% is caused by analytical measurement deviation and experimental errors. This means that there has been achieved an enrichment of deuterium in the respective position and that hydrogen has been replaced. The respective enrichment may be performed by chemical reaction in that one uses deuterated starting materials in chemical reactions or that an hydrogen/deuterium exchange has been performed by mixing respective compounds.

"Deuterated" is therefore not related to any naturally occurring deuterium in hydrogen compounds. As it is known, deuterium is present in hydrogen in natural abundance to an extend of 0.015 mol%. Any abundance or enrichment that is greater than 0.02 mol% is understood as being "deuterated" in the sense of this present invention.

The problem is solved, according to the present invention, by providing a pharmaceutical composition which comprises a dopamine agonist and a L-DOPA derivative in combination in form of a liquid preparation.

It is preferred that the pharmaceutical composition, according to the present invention, further comprises pharmaceutically acceptable adjuvants and additives from the group of solvents, sugars or pH regulators.

Preferred is also a pharmaceutical composition, according to the present invention, wherein the dopamine agonist is selected from the group comprising apomorphine, ropinirole, rotigotine, pramipexole, and piribedile.

Preferred is also a pharmaceutical composition, according to the present invention, wherein the L-DOPA derivative is selected from L-DOPA, selectively and/ or partially deuterated L-DOPA derivatives, as well as physiologically acceptable salts of the aforementioned L-DOPA derivatives.

It is also preferred that the pharmaceutical composition, according to the present invention, contains one or more deuterated derivatives of L-DOPA as well as physiologically acceptable salts thereof.

The pharmaceutical composition, according to the present invention, is further characterized in that the deuterated derivative of L-DOPA is selected from the group consisting of S/S-2-amino-2,3-dideutero-3-(3,4dihydroxyphenyl) propionic acid (α,β-D2-L-DOPA), L-2-Amino-2,3,3trideutero-3-(3,4-dihydroxyphenyl) propionic acid (α,β,β-D3-L-DOPA) or L-2-Amino-2,3,3*-trideutero-3-(3,4-dihydroxyphenyl) propionic acid (α,β,β*-D3-L-DOPA) and the physiological acceptable salts thereof.

Especially preferred according to the present invention is that L-2-Amino2,3,3*-trideutero-3-(3,4-dihydroxyphenyl) propionic acid (α,β,β*-D3-L-DOPA) is selected, wherein 3* indicates that the deuterium enrichment in one β-position is about 90 mol%. The other positions carrying deuterium are completely deuterated and show a deuterium enrichment of at least 98 mol%. The preparation of these deuterated L-DOPA derivatives can be performed by mixing compounds with a certain deuterium enrichment with compounds which have only hydrogen or only a highly enriched (>98 % D) deuterium substitution at a certain position. By mixing at least two compounds any required enrichment level of deuterium at any position can be obtained.

Preferred is the pharmaceutical composition, according the present invention, which additionally contains at least one DOPA decarboxylase inhibitor.

Especially preferred is the pharmaceutical composition, according to the present invention, which is characterized in that the DOPA decarboxylase inhibitor is selected from the group of (-)-L-α-hydrazino-3,4-dihydroxy-α-methylhydrocinnamic acid (carbidopa), D,L-serine 2-(2,3,4-trihydroxybenzyl) hydrazide (benserazide), L-serine-2-(2,3,4-trihydroxybenzyl) hydrazide, glycine-2-(2,3,4-trihydroxybenzyl) hydrazide or L-tyrosine-2-(2,3,4-trihydroxybenzyl) hydrazide and the physiological acceptable salts thereof.

Preferred is the pharmaceutical composition, according to the present invention, wherein the dopamine agonist is apomorphine and wherein the amount of apomorphine is between 2-30 mg/ml.

Further preferred is the pharmaceutical composition, according to the present invention, wherein the amount of apomorphine is 5 mg/ml.

Preferred is the pharmaceutical composition, according to the present invention, wherein the L-DOPA derivative is L-DOPA and wherein the amount of L-DOPA is between 5-50 mg/ml.

Especially preferred is the pharmaceutical composition, according to the present invention, wherein the amount of L-DOPA is between 10-15 mg/ml.

Preferred is the pharmaceutical composition, according to the present invention, wherein the amount of carbidopa is between 0.5-10 mg/ml.

Further preferred is the pharmaceutical composition, according to the present invention, wherein the amount of carbidopa is between 2-3 mg/ml.

A further object of the present invention is a method for the preparation of the pharmaceutical composition by mixing a dopamine agonist and a L-DOPA derivative in a defined ratio to each other.

Another object is the use of the pharmaceutical composition, according to the present invention, in addition to pharmaceutically acceptable adjuvants and additives, for the treatment of Parkinson's disease, of restless leg syndrome, of dystonia, for inhibiting prolactin secretion, for stimulating the release of growth hormones, for the treatment of neurological symptoms of chronic manganese intoxication, of amyotrophic lateral scleroses and of multiple system atrophy.

Preferred is the use of the pharmaceutical composition, according to the present invention, characterized in a form of a parental application selected from infusions, injections or an application via a gastric tube.

The production of the pharmaceutical preparations according to the invention is known in the art, and is described in handbooks known to the person skilled in the art, for example, Hager's Handbuch [Handbook] (5th ed.) 2, 622-1045; List et al., Arzneiformenlehre [Instructions for Drug Forms], Stuttgart: Wiss. Verlagsges. 1985; Sucker et al., Pharmazeutische Technologie [Pharmaceutical Technology], Stuttgart: Thieme 1991; Ullmann's Enzyklopädie [Encyclopedia] (5th ed.) A 19, 241-271; Voigt, Pharmazeutische Technologie [Pharmaceutical Technology], Berlin: Ullstein Mosby 1995.

Thus, a preferred embodiment of the present invention is directed to a pharmaceutical composition comprising apomorphine, L-DOPA and DOPA decarboxylase inhibitor in a defined ratio to each other.

The use of a pharmaceutical composition according to the invention instead of the currently used apomorphine infusion with concomitant oral levodopa administration allows up to 40% reduction of the LED.

Besides using L-DOPA in the present invention, the pharmaceutical composition my also comprise a deuterated form of L-DOPA.

The preparation of deuterium enriched catecholamine derivatives (L-DOPA derivatives) is known from WO-A 2004/056724 and WO-A 2007/093450. In there, the preparation of selectively deuterated DOPA derivatives is disclosed that have a deuterium enrichment in the respective position within the molecule of at least 98 %.

For the production of the pharmaceutical composition according to the present invention the following usually pharmaceutically acceptable adjuvants and additives can be used, selected from the group of solvents/solubilizers, sugars, pH regulators and antioxidants.

Solvents can be used for the preparation of the pharmaceutical composition in order to obtain the required injectability criteria. Solvents can be selected from alcohols such as ethanol, isopropanol, methanol or n-propanol. Solvents can also be selected from polyols such as propyleneglycol, glycerol, mannitol, maltitol or from cyclodextrin derivatives such as sulfobutylether β cyclodextrin or hydroxypropyl β cyclodextrin. Further solvents can be selected from the group of polyethers.

Surfactants could also be used for the preparation of the pharmaceutical composition according to the present invention. Surfactants can be used from the group of polyoxyethylene sorbitan fatty acid esters such as polysorbate 80 or polysorbate 20.

Further physiologically acceptable sugars such as dextran, mannitol or glucose can be used for the preparation of the pharmaceutical composition according to the invention.

The pH of the pharmaceutical composition plays a crucial role for the preparation. Levodopa is poorly dissolved at neutral pH. Best solubility of levodopa is achieved at a lower pH between 3 and 6. Whereas a pH below 4 could cause blood vessel irritations and thrombophlebitis. Therefore a pH in the range of 4 to 6 is preferred for the pharmaceutical composition according to the present invention whereas a range of 4.5 to 5.5 is even more preferred. In order to achieve the required pH values, pH regulators are used during the preparation of the pharmaceutical composition. PH regulators can be selected from the usual physiologically acceptable inorganic and organic acids such as hydrochloric acid, hydrobromic acid, phosphoric acid, sulfuric acid, oxalic acid, maleic acid, fumaric acid, lactic acid, tartaric acid, malic acid, citric acid, salicylic acid, adipic acid and benzoic acid. According to the invention, weak acids such as hydrochloric acid and acetic acid are especially preferred for the production of the pharmaceutical composition.

Further usually pharmaceutically acceptable adjuvants and additives which can be used are antioxidants. Antioxidants can be selected from the group of acids and their salts, vitamins and vitamin derivatives, amino acids, sulfites and free phenolic radical scavengers as well as thioctic acid.

The pharmaceuticals of the invention are produced, in the known way and with suitable dosage, with the usual solid or liquid vehicle substances or dilution agents and the usually used pharmaceutical-technical adjuvants corresponding to the desired type of application.

The subject of the present invention is also a pharmaceutical composition for subcutaneous, intravenous or intramuscular application.

Solutions or suspensions containing the active substances used according to the invention may additionally contain suspension adjuvants such as sodium carboxymethylcellulose or preservatives such as p-hydroxybenzoate.

Further in addition to the traditional application ways, the pharmaceutical composition according to the present invention can also be used in infusion pumps for subcutaneous or intrathekal application. Different infusion systems have been described in the literature relaying on two different ways of application. Either the pharmaceutical composition can be used as infusion solution in a cassette or in a bottle connected with an adapter to the pump system.

The pharmaceutical composition according to the invention could also be used in the gastric pumps known in the art.

The following examples shall explain the present invention. The examples shall be understood only as one preferred embodiment of the invention. It is not intended to limit the present invention to the scope of the given examples.

The following examples provide possible recipes for an injectable solution of the pharmaceutical composition according to the present invention with and without the additional use of carbidopa.

### Example 1

### Preparation of a pharmaceutical composition containing levodopa (L-DOPA) and apomorphine.

### Stock solution 1:

Levodopa 50 mg/ml and Apomorphine 25 mg/ml

| **Substance** | **Weight** |
|---|---|
| Levodopa | 5 g |
| Hydrochloric acid | 15.5 g |
| Sodium pyrosulfite | 0.5 g |
| Apomorphine | 2.5 g |
| Propyleneglycol | 30 g |
| Polysorbate 80 | 0.3 g |
| Water | Filed up to 100 ml |

Levodopa is weighed and dissolved in a beaker containing a HCL solution while stirring. The sodium pyrosulfite is weighed in a small beaker and dissolved by adding 2 ml of sterile water. The dissolved pyrosulfite solution is taken up by a pipette and added to the solution containing levodopa.
In the meanwhile propylene glycol and polysorbate 80 are weighed and dissolved with water in a medium-sized beaker. Apomorphine is added to the propylene glycol / polysorbate 80 solution after adjusting the pH. The solution containing apomorphine is stirred on a magnetic stirrer until all substances are completely dissolved. After apomorphine is dissolved the solution is added to the beaker containing the levodopa solution.
The solution is filled up to 100 ml with sterile water and stirred on a magnetic stirrer. The combined solutions are further stirred until a completely homogenized solution is prepared.

### Solution 1:

Levodopa 10 mg/ml and Apomorphine 5 mg/ml

| **Substance** | **Weight** |
|---|---|
| Levodopa and apomorphine | 20 ml of the stock solution 1 |
| TRIS Buffer | 3 ml |
| Glucose | 25 mg/ml add to 100 ml |

For a ready-to-use solution in order to perform injections or use the solution in infusion pumps, TRIS buffer is added to the solution containing levodopa and apomorphine. Glucose is slowly added under stirring, up to a total volume of 100 ml. The pH is checked before the final solution is sterile filtered and used.

Instead of using the solvents propylenglycol (30%) and polysorbate (0.3%) to dissolve apomorphine, either the solvent sulfobuthylehter β cyclodextrin (20%) or the solvent hydroxypropyl β cyclodextrin (20%) can be used as well.

### Example 2

### Preparation of a pharmaceutical composition containing levodopa and apomorphine and carbidopa.

### Stock solution 2:

Levodopa 50 mg/ml, Apomorphine 25 mg/ml and Carbidopa 10 mg/ml

| **Substance** | **Weight** |
|---|---|
| Levodopa | 5 g |
| Hydrochloric acid | 15.5 g |
| Sodium pyrosulfite | 0.5 g |
| Apomorphine | 2.5 g |
| Propyleneglycol | 30 g |
| Polysorbate 80 | 0.3 g |
| Carbidopa | 1 g |
| Water | Filed up to 100 ml |

Levodopa is weighed and dissolved in a beaker containing a HCL solution while stirring. The sodium pyrosulfite is weighed in a small beaker and dissolved by adding 2 ml of sterile water. The dissolved pyrosulfite solution is taken up by a pipette and added to the solution containing levodopa.
In the meanwhile propylene glycol and polysorbate 80 are weighed and dissolved with water in a medium-sized beaker. Apomorphine is added to the propylene glycol / polysorbate 80 solution after adjusting the pH. The solution containing apomorphine is stirred on a magnetic stirrer until all substances are completely dissolved. After apomorphine is dissolved the solution is added to the beaker containing the levodopa solution. The combined solutions are further stirred until a completely homogenized solution is prepared. The pH is checked.

Carbidopa is weighed in dissolved in a beaker containing sterile water and stirred until completely dissolved. Heating might be required.
Afterwards the carbidopa solution is added to the solution containing levodopa and apomorphine.
The solution is filled up to 100 ml with sterile water and stirred on a magnetic stirrer. The final solution is filtered before use.

Instead of using the solvents propylenglycol (30%) and polysorbate (0.3%) to dissolve apomorphine, either the solvent sulfobuthylehter β cyclodextrin (20%) or the solvent hydroxypropyl β cyclodextrin (20%) can be used as well.

### Solution 2:

Levodopa 10 mg/ml, Apomorphine 5 mg/ml and Carbidopa 2 mg/ml

| **Substance** | **Weight** |
|---|---|
| Levodopa, apomorphine and carbidopa | 20 ml of the 50 mg/ml stock solution2 |
| TRIS Buffer | 3 ml |
| Glucose | 25 mg/ml add to 100 ml |

For a ready-to-use solution in order to perform injections or use the solution in infusion pumps, TRIS buffer is added to the solution containing levodopa, apomorphine and carbidopa. Glucose is slowly added under stirring, up to a total volume of 100 ml. The final solution is sterile filtered before use.

### Example 3

### Preparation of a pharmaceutical composition comprising a deuterated form of L-DOPA namely S/S-2-amino2,3-dideutero-3-(3,4-dihydroxyphenyl) propionic acid (α,β-D2-L-DOPA), apomorphine and carbidopa.

The stock solution 3 is prepared as the stock solution 2 described in example 2 containing 50 mg/ml α,β,β*-D3-L-DOPA, 25 mg/ml apomorphine and 10 mg/ml carbidopa.
Apomorphine is dissolved in the solvent hydroxypropyl β cyclodextrin (20%) instead of propylenglycol and polysorbate 80 as described in example 2 before being mixed with the solution containing the dissolved α,β-D2-L-DOPA.

Afterwards a working solution 3 is diluted having an end concentration of 10 mg/ml α,β-D2-L-DOPA, 5 mg/ml apomorphine and 2 mg/ml carbidopa. The diluted solution is prepared as described in example 2 and also sterile filtered before use.

### Example 4

### Preparation of a pharmaceutical composition containing apomorphine and carbidopa together with a deuterated form of L-DOPA which is not completely deuterated at one position indicated by an asterix (β*).

The preparation of the stock solution 4 containing 50 mg/ml L-2-Amino-2,3,3-trideutero-3-(3,4-dihydroxyphenyl) propionic acid (α,β,β*-D3-L-DOPA), 25 mg/ml apomorphine and 10 mg/ml carbidopa is in accordance to the description found in example 2. The only difference is the solvent used. Instead of using propylenglycol and polysorbate 80 as described in example 2, sulfobutylether β cyclodextrin is used to dissolve apomorphine.

The α,β,β*-D3-L-DOPA has a deuterium enrichment of 90 % in β_{R} position. α,β,β*-D3-L-DOPA is obtained by mixing 10 mol% L-2-Amino-2,3(S)-dideutero-3-(3,4-dihydroxyphenyl) propionic acid with 90 mol% L-2-Amino-2,3,3-trideutero-3-(3,4-dihydroxyphenyl) propionic acid (deuterium enrichment >98 % in all three positions).

Before using the prepared pharmaceutical composition, a working solution 4 is diluted from stock solution 4 having an end concentration of 10 mg/ml α,β,β*-D3-L-DOPA, 5 mg/ml apomorphine and 2 mg/ml carbidopa. The diluted solution is prepared as described in example 2 and also sterile filtered before use.

## Claims

1. A pharmaceutical composition comprising
i) a dopamine agonist and
ii) a L-DOPA derivative
in combination in form of a liquid preparation.

2. The pharmaceutical composition, according to claim 1, further comprising pharmaceutically acceptable adjuvants and additives from the group of solvents, sugars or pH regulators.

3. The pharmaceutical composition, according to claim 1 or 2, wherein the dopamine agonist is selected from the group comprising apomorphine, ropinirole, rotigotine, pramipexole, and piribedile.

4. The pharmaceutical composition, according to a least one of the preceding claims, wherein the L-DOPA derivative is selected from L-DOPA, selectively and/ or partially deuterated L-DOPA derivatives, as well as physiologically acceptable salts of the aforementioned L-DOPA derivatives.

5. The pharmaceutical composition, according to a least one of the preceding claims, which contains one or more deuterated derivatives of L-DOPA as well as physiologically acceptable salts thereof.

6. The pharmaceutical composition, according to claim 5, further **characterized in that** the deuterated derivative of L-DOPA is selected from the group consisting of S/S-2-amino-2,3-dideutero-3-(3,4-dihydroxyphenyl) propionic acid (α,β-D2-L-DOPA), L-2-Amino-2,3,3-trideutero-3-(3,4-dihydroxyphenyl) propionic acid (α,β,β-D3-L-DOPA) or L-2-Amino-2,3,3*-trideutero-3-(3,4-dihydroxyphenyl) propionic acid (α,β,β*-D3-L-DOPA), wherein 3* or β* indicates that the deuterium enrichment in one β-position is about 90 mol% and the physiological acceptable salts thereof.

7. The pharmaceutical composition, according to at least one of the preceding claims, which additionally contains at least one DOPA decarboxylase inhibitor.

8. The pharmaceutical composition, according to claim 7, **characterized in that** the DOPA decarboxylase inhibitor is selected from the group of (-)-L-α-hydrazino-3,4-dihydroxy-a-methylhydrocinnamic acid (carbidopa), D,L-serine 2-(2,3,4-trihydroxybenzyl) hydrazide (benserazide), L-serine-2-(2,3,4-trihydroxybenzyl) hydrazide, glycine-2-(2,3,4-trihydroxybenzyl) hydrazide or L-tyrosine-2-(2,3,4-trihydroxybenzyl) hydrazide and the physiological acceptable salts thereof.

9. The pharmaceutical composition, according to at least one of the preceding claims, wherein the dopamine agonist is apomorphine and wherein the amount of apomorphine is between 2-30 mg/ml, preferred 5 mg/ml.

10. The pharmaceutical composition, according to at least one of the preceding claims, wherein the L-DOPA derivative is L-DOPA and wherein the amount of the L-DOPA is between 5-50 mg/ml, preferred 10-15 mg/ml.

11. The pharmaceutical composition, according to claim 7, wherein the DOPA decarboxylase inhibitor is carbidopa and wherein the amount of carbidopa is between 0.5-10 mg/ml, preferred 2-3 mg/ml.

12. A method for the preparation of the pharmaceutical composition, according to at least one of the preceding claims, by mixing at least one dopamine agonist and at least one L-DOPA derivative in a defined ratio to each other.

13. Use of the pharmaceutical composition, according to at least one of the preceding claims, in addition to pharmaceutically acceptable adjuvants and additives, for the treatment of Parkinson's disease, of restless leg syndrome, of dystonia, for inhibiting prolactin secretion, for stimulating the release of growth hormones, for the treatment of neurological symptoms of chronic manganese intoxication, of amyotrophic lateral scleroses and of multiple system atrophy.

14. Use of the pharmaceutical composition, according to at least one of the preceding claims, **characterized in** a form of a parental application selected from infusions, injections or an application via a gastric tube.
